Europäisches Patentamt

European Patent Office

Office européen des brevets

(19)

(11) Publication number: 0 354 597
A2

(12) EUROPEAN PATENT APPLICATION

(21) Application number: 89118782.5

(22) Date of filing: 31.05.85

(51) Int. Cl.4: A61M 1/36 , A61M 5/168

(30) Priority: 18.06.84 SE 8403244

(43) Date of publication of application:
14.02.90 Bulletin 90/07

(60) Publication number of the earlier application in
accordance with Art.76 EPC: 0 165 519

(84) Designated Contracting States:
BE CH DE FR GB IT LI NL

(71) Applicant: Gambro Lundia AB
Box 10101
S-220 10 Lund(SE)

(72) Inventor: Dahlberg, Bengt Ake Gustav
Dörröd 7
S-240 14(SE)
Inventor: Jeppsson, Jan-Bertil
Västkustvägen 86
S-234 00 Lomma(SE)
Inventor: Olsson, Lars Ingvar
Ekebergslundsvägen 8
S-292 02 Asarum(SE)
Inventor: Simonsson, Benny Tord
Drömvägen 6
S-261 61 Landskrona(SE)

(74) Representative: Boberg, Nils Gunnar Erik
Gambro AB Patent Department Box 10101
S-220 10 Lund(SE)

(54) Use of a drip and/or expansion chamber.

(57) Use of a drip and/or expansion chamber comprising a gas-permeable but liquid-tight membrane (46d) controlling the fluid level, the dry side of the membrane being connected to the outer atmosphere or a pressure-controlling device via a duct (48d).

The use is characterized in that said drip and/or expansion chamber is being utilized so that said duct points vertically downwards so that any condensate formed runs away from the membrane and not towards the same.

## USE OF A DRIP AND/OR EXPANSION CHAMBER

### TECHNICAL FIELD

The present invention relates to the use of a drip and/or expansion chamber, said chamber comprising a gas-permeable but liquid-tight membrane controlling the fluid level, the dry side of the membrane being connected to the outer atmosphere or a pressure controlling device via a duct.

The invention is in the first place intended to be used in the blood filtering system disclosed in EP 0 165 519. It will be clear, however, to those versed in the art that the invention can also be applied with advantage in for instance other extra-corporeal systems for treating blood or blood plasma.

Due to the fact that the present application is a divisional application of the above mentioned application EP 0 165 519 the contents of said application is included in the present application by reference.

### BACKGROUND ART

A drip chamber of the above mentioned art is disclosed in for instance the European patent application EP 0 062 913. The device described in said publication has, however, the drawback that possible created condensate can flow downwardly and block up the gas-permeable but liquid-tight membrane.

### DISCLOSURE OF INVENTION

The above mentioned problem is solved according to the present invention by the use of a drip and/or expansion chamber of the above-defined kind in such a way that said duct points vertically downwards so that any condensate formed runs away from the membrane and not towards the same.

When the chamber is used as a drip chamber it has to be provided with an inlet arranged at its upper end and it is preferable also provided with means for the prevention of froth formation or for the breaking down of any froth already formed between the inlet and the membrane.

The membrane is preferably arranged horizontally in a disc filter which is connected via a duct to a gas outlet arranged at the bottom of the chamber. The liquid passing chamber is preferably leaving it through an outlet which also is arranged at the bottom of the chamber.

### BRIEF DESCRIPTION OF DRAWING

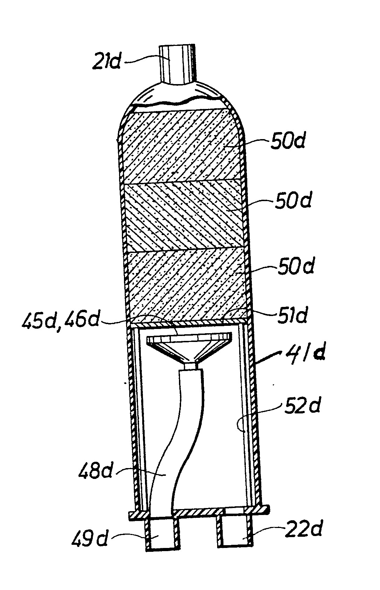

The drawing is showing a drip and/or expansion chamber which is intended to be used in accordance with the present invention.

### PREFERRED EMBODIMENT OF THE INVENTION

A preferred embodiment of the invention is shown on the drawing. The chamber, here shown as an example, consists of a housing 41d with an inlet 21d and an outlet 22d. In the chamber a disc filter 45d is arranged carrying a horizontal membrane 46d. The disc filter is connected to an outlet 49d via a tube or duct 48d.

The chamber includes furthermore three filters 50d which are intended to prevent froth formation or for the breaking down of any froth already formed. For this purpose the filters are made of a foam material such as polyurethane, and impregnated with a froth breaking down material such as silicone. The filters are carried by a plate 51d arranged on supporting legs 52d.

If the chamber is used as described in the above mentioned publication EP 0 165 519, i e for the equalizing of the pressure in a filtrate duct, the filtrate is fed to the chamber through the inlet 21d and taken away via the outlet 22d. Any gas leaving the filtrate is removed via the membrane 46d and the tube 48d to the outlet 49d. Should the liquid level reach above the membrane 46d, it will be pressed slowly downwardly due to incoming gas bubbles until the membrane is free again. Should the liquid level instead be situated below the membrane 46, the gas can freely flow out through the membrane and the liquid level will rise. In order to prevent that drips are accumulated on the membrane it may possibly be arranged in a slightly oblique position.

Possibly formed filtrate froth is broken down by means of the polyurethane foam which is impregnated with a silicone oil. For practical reason the filter has been designed as three discs arranged on top of one another. Thank to the plate 51d the filtrate is brought to follow the inner wall of the chamber even if the liquid level should be below the plate 51d.

An advantage with the design shown is that the tube 48d normally will be situated within the liquid reducing the risk for condensate formation. However, if condensate should be formed, it can not flow against the membrane and block up said membrane but will instead flow in the direction away from the membrane.

Naturally the invention is not limited exclusively to the use of the embodiment described above, but can be varied within the scope of the following claims. Thus, for example, the design of the chamber may be varied within wide limits.

## Claims

1. Use of a drip and/or expansion chamber, said chamber comprising a gas-permeable but liquid-tight membrane (46d) controlling the fluid level, the dry side of the membrane being connected to the outer atmosphere or a pressure-controlling device via a duct (48d), **characterized** in that said drip and/or expansion chamber being utilized so that said duct points vertically downwards so that any condensate formed runs away from the membrane and not towards the same.

2. The use according to claim 1 of a drip chamber comprising an inlet (21d) arranged at its upper end, **characterized** in that it is provided with means (50d) for the prevention of froth formation or for the breaking down of any froth already formed between the inlet (21d) and the membrane (46d).

3. The use according to claim 2, **characterized** in that the membrane (46d) is arranged horizontally in a disc filter (45d) which is connected via a duct (48d) to a gas outlet (49d) arranged at the bottom of the chamber.

4. The use according to claim 2, **characterized** in that the outlet (22d) of the drip chamber is arranged at the bottom of the chamber.

21d

50d

50d

50d

45d,46d

51d

41d

52d

48d

49d

22d